(19) Europäisches Patentamt
European Patent Office
Office européen des brevets

(11) EP 1 362 574 B1

(12) **EUROPEAN PATENT SPECIFICATION**

(45) Date of publication and mention of the grant of the patent:
**14.03.2007 Bulletin 2007/11**

(51) Int Cl.:
***A61Q 5/04*** *(2006.01)* ***A61K 8/42*** *(2006.01)*

(21) Application number: **03010303.0**

(22) Date of filing: **07.05.2003**

(54) **Method of treating hair**

Haarbehandlungsverfahren

Procédé pour traiter les cheveux

(84) Designated Contracting States:
**DE FR GB**

(30) Priority: **10.05.2002 JP 2002135415**

(43) Date of publication of application:
**19.11.2003 Bulletin 2003/47**

(73) Proprietor: **KAO CORPORATION**
**Tokyo 103-8210 (JP)**

(72) Inventor: **Hirano, Yuji**
**Sumida-ku,**
**Tokyo 131-8501 (JP)**

(74) Representative: **HOFFMANN EITLE**
**Patent- und Rechtsanwälte**
**Arabellastrasse 4**
**81925 München (DE)**

(56) References cited:
**EP-A- 1 166 766**
**WO-A-90/14429**
**DE-A- 19 907 381**
**DE-U- 29 712 458**
**US-A- 5 628 991**
**US-A- 5 756 784**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 362 574 B1

## Description

### TECHNICAL FIELD

The present invention relates to hair processing compositions which cause little hair damage and do not remove the moisture from the hair.

### BACKGROUND ART

It is the common practice to adopt permanent waving or permanent straightening treatment in order to set the hair to a desired shape and retain the shape as long as possible. In such treatment, the hair is fixed to a desired shape by applying a first part composition containing a reducing substance or alkali agent such as thioglycolate or cysteine to the hair to cause cleavage of a cystine bond therein, and swell and soften the hair, and then applying the second part composition containing an oxidizing agent such as bromate or hydrogen peroxide to the hair to re-establish the cystine bond. In the case of permanent waving, the desired shape is available by winding the hair onto a rod or the like before or after application of the first part composition, while in the case of permanent straightening, the hair is straightened by a comb or the like after application of the first part composition. The hair treated with a perming agent is however liable to be damaged by daily hair care behaviors (shampooing, or combing or blowing for styling). More specifically, it is known that split or cut hair generates at the hair end, the hair becomes dry to the touch, or the shape fixed by perming treatment does not last long enough.

With a view to preventing such hair damage caused by permanent waving or permanent straightening treatment, a method of incorporating an oil substance such as silicone or paraffin, conditioning polymer, or protein component (for example, protein hydrolysate) into a perming agent or into a hair care agent to be used after the perming treatment has been proposed. However, such method does not always bring about sufficient effects.

### DISCLOSURE OF THE INVENITON

An object of the present invention is therefore to provide a method of permanent waving or permanent straightening hair which causes less hair damage and does not impair the moisture intrinsic to the hair.

The present inventors have found that hair damage can be decreased markedly by incorporating into a hair processing composition a diamide compound represented by the following formula (1):

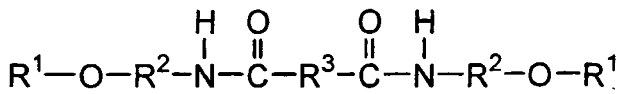

(1)

wherein, $R^1$ represents a linear or branched $C_{1-12}$ hydrocarbon group which may be substituted by a hydroxy and/or alkoxy group, $R^2$ represents a linear or branched divalent $C_{1-5}$ hydrocarbon group, and $R^3$ represents a linear or divalent branched $C_{1-22}$ hydrocarbon group.

In the present invention, there is thus provided a method of permanent waving or permanent straightening hair as defined by claim 1.

### BEST MODE FOR CARRYING OUT THE INVENTION

In the formula (1) representing the diamide compound serving as Component (A) in the present invention, preferred as $R^1$ are linear or branched $C_{1-12}$ alkyl groups which may be substituted by 1 to 3 substituents selected from a hydroxy group and $C_{1-6}$ alkoxy groups. Among them, unsubstituted $C_{1-12}$ alkyl groups, and $C_{2-12}$ alkyl groups each substituted by one or two hydroxy groups, by one $C_{1-6}$ alkoxy group, or by one hydroxy group and one $C_{1-6}$ alkoxy group are more preferred.

In the formula (1), preferred as $R^2$ are linear or branched $C_{2-5}$, particularly $C_{2-3}$ alkylene groups.

In the formula (1), preferred as $R^3$ are linear or branched divalent $C_{2-22}$ hydrocarbon groups, among which linear or branched $C_{11-22}$ alkylene groups and alkenylene groups having 1 to 4 double bonds are particularly preferred.

Particularly preferred diamide compounds as Component (A) include compounds having, as $R^1$, $R^2$ and $R^3$ in the formula (1), the above-exemplified respective preferred groups in combination. Specific examples of the particularly preferred diamide compounds (1) are shown below:

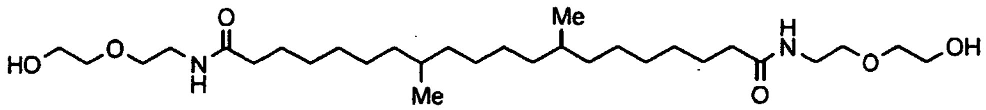
(A)

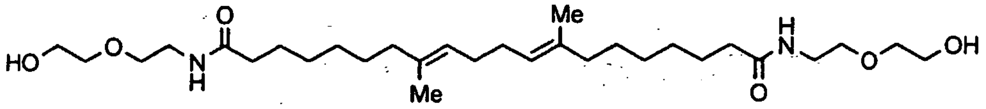
(B)

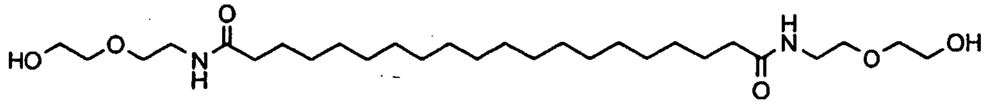
(C)

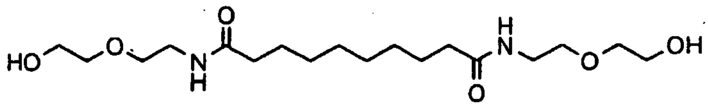
(D)

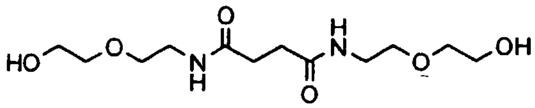
(E)

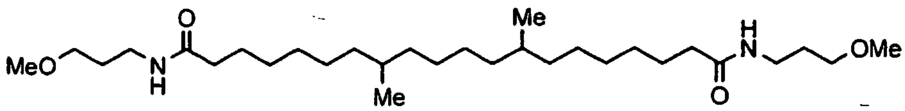
(F)

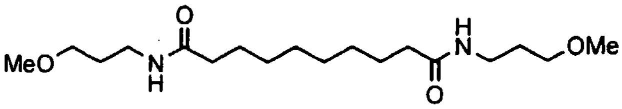
(G)

The diamide compounds (1) can be prepared by a known amide synthesizing process. For example, the intended diamide compound (1) can be prepared efficiently at a low cost by condensing the corresponding dicarboxylic acid (2) or reactive derivative thereof (ester, acid halide, acid anhydride, or the like) with an amine (3) in accordance with the following reaction scheme (International Publication No. 00/61097 brochure):

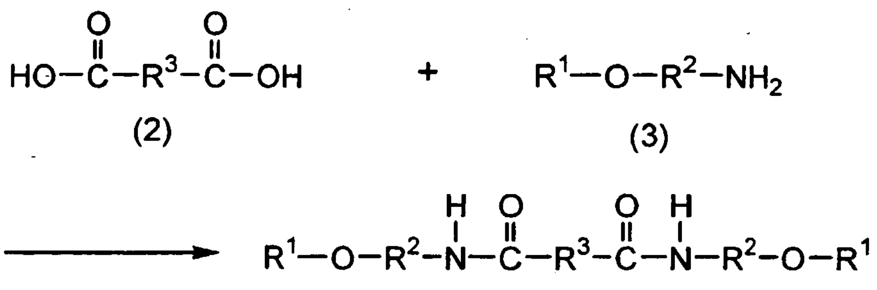
(2) (3) (1)

The hair processing composition used according to the present invention is a two-part system one, wherein the diamide compound (1) serving as Component (A) can be incorporated in any one of or both of the first part and the second part. As the diamide compound (1) serving as Component (A), two or more can be used in combination. For

sufficiently suppressing the hair damage and at the same time, avoiding deterioration in feeling upon use, the content of the diamide compound is preferably from 0.01 to 30 wt.%, more preferably from 0.1 to 20 wt.%, especially preferably from 0.5 to 15 wt.% based on the composition just before use.

Examples of the reducing agent to be used in the present invention as Component (B) include thioglycolic acid and salts or esters thereof; thio acids such as thiolactic acid, thiomalic acid, thiotartaric acid, and 3-mercaptopropionic acid, and esters thereof; cysteines such as cysteine, homocysteine, cysteamine and N-acyl cysteine, and salts or esters thereof; mercaptocarboxylic amides such as mercaptoacetamide, mercaptopropionamide and cysteine amide, and N-substituted derivatives thereof; thioglyceryl alkyl ethers; mercaptoalcohols such as 2-mercaptoethanol, thioglycerol, and 3-alkoxy-1-mercapto-2-propanol; thiosaccharides such as 1-thio-β-D-glucose; and keratin reducing substances such as sulfites and bisulfites. The salts of these substances include sodium salts, potassium salts, ammonium salts, and ammonium salts of an organic amine (monoethanolamine, etc). Among them, thioglycolic acid, thio acid, cysteine and N-acyl cysteine are preferred from the viewpoints of hairstyle changing effects (permanent waving effects, curl straightening effects) and safety. Two or more of the above-exemplified reducing agents may be used in combination. In the hair processing composition used according to the present invention the reducing agent is incorporated in the first part and its content ranges from 0.1 to 30 wt.% based on the total weight of the first part, with a range of from 1 to 20 wt.% being particularly preferred.

Examples of the oxidizing agent serving as component (B) in the present invention include hydrogen peroxide, urea peroxide, bromates of an alkali metal and peracid salts of an alkali metal (perbromates, persulfates, perborates, etc.). Two or more of these oxidizing agents may be used in combination. The oxidizing agent is incorporated in the second part and its content preferably falls within a range of from 1 to 20 wt.% based on the total weight of the second part composition, with a range of from 1 to 10 wt.% being especially preferred.

To the hair processing composition used according to the present invention, components ordinarily employed for the same purpose can be added further as desired. Examples of such optional components include alkali agents, surfactants, oil substances, solubilizing agents, buffers, stabilizers, perfumes, colorants, antiseptics, pH regulators, thickeners, hair protecting agents, UV protectives, anti-inflammatories, humectants, feel improvers, astringents, chelating agents and hair growth promoting components.

The hair processing composition used according to the present invention can be prepared in a conventional manner and it can be provided in the desired form such as lotion, cream, emulsion, gel and aerosol foam. In addition, the hair processing composition used according to the present invention is a two-part system composition having a first part composed mainly of a reducing agent and a second part composed mainly of an oxidizing agent.

**- Examples -**

Examples 1 to 4, and Comparative Example 1

The first part and second part compositions of a perming agent shown in Tables and 2, respectively were prepared and "prevention of moisture loss" and "ratio of generated split hair" after the hair was treated with each agent were evaluated to study the effect of the invention. The results are shown in Table 3.

• Preparation process:

Components (1), (6) and (7) as shown in Table 1 were mixed. After the resulting mixture was heated to 60°C and dissolved uniformly, it was gradually added to a mixture of Components (2) to (5) and (8) heated to 60°C. After 20 minutes stirring, the mixture was cooled back to room temperature, whereby the first part composition was obtained.

Components (1), (3) and (4) as shown in Table 2 were mixed and the resulting mixture was heated to 60°C to make it uniform. The mixture was then added gradually to a mixture of Components (2) to (5) heated to 60°C. After 20 minutes stirring, the resulting mixture was cooled back to room temperature, whereby the second part composition was obtained.

Treating process:

A 16-cm hair bundle made of 100 hairs (about 0.1 g) of a Japanese female was used as a tress for evaluation. The first part composition was applied to the tress at a hair:first part composition bath ratio (weight ratio) of 1:1 and the tress was wetted with the composition. The resulting tress was allowed to stand at room temperature for 20 minutes. The second part composition was applied to the tress at a hair:second part composition bath ratio (weight ratio) of 1:1 and the tress was wetted with the composition. After allowing to stand at room temperature for 10 minutes, the tress was rinsed to remove the compositions, shampooed once with a commercially available shampoo ("Essential Damage Care Shampoo", product of Kao Corporation) and then dried with a hair dryer. As the first part and the second part

compositions, a combination as shown in Table 3 was employed.

• Evaluation method and criteria

(1) Prevention of moisture loss

The tress for evaluation after the above-described treatment was subjected to organoleptic evaluation by a panel of 10 experts to examine the moisture loss. Average evaluation scores in accordance with the below-described criteria were judged as A, B and C when the average scores were 2.4 or greater, 1.6 to 2.3 and 1.0 to 1.5, respectively.

<Evaluation criteria>

There is no feel of moisture loss: 3
There is some feel of moisture loss: 2
There is a feel of moisture loss: 1

(2) Ratio of generated split hair

A brushing stimulus was applied to the treated tress With a rotating hairbrush driven by a motor at a rotation speed of 100 times/minute for 60 minutes. The number (D) of split or cut hairs was then counted. The split hair - generation ratio was determined from the following formula:

$$\text{Split hair-generation ratio (\%)} = D \div 100 \times 100 = D$$

Table 1

| | First Part A | First Part B | First Part C |
|---|---|---|---|
| (1) Diamide compound (A) | 1.0 | 2.0 | - |
| (2) Ammonium thioglycolate (50 wt.%) | 12.0 | 12.0 | 12.0 |
| (3) Disodium Edetate | 0.5 | 0.5 | 0.5 |
| (4) Monoethanolamine | q.s. (pH 9) | q.s. (pH 9) | q.s. (pH 9) |
| (5) Ammonium bicarbonate | 2.5 | 2.5 | 2.5 |
| (6) Propylene glycol | 15.0 | 15.0 | 15.0 |
| (7) Polyoxyethylene (9) tridecyl ether | 1.0 | 1.0 | 1.0 |
| (8) Purified water | Balance | Balance | Balance |
| Total | 100 wt.% | 100 wt.% | 100 wt.% |

Table 2

| | Second Part A | Second Part B |
|---|---|---|
| (1) Diamide compound (A) | 1.0 | - |
| (2) Sodium bromate | 7.0 | 7.0 |
| (3) Propylene glycol | 15.0 | 15.0 |
| (4) Polyoxyethylene (9) tridecyl ether | 1.0 | 1.0 |
| (5) Purified water | Balance | Balance |
| Total | 100 wt.% | 100 wt.% |

Table 3

| | | Example 1 | Example 2 | Example 3 | Example 4 | Com. Ex. 1 |
|---|---|---|---|---|---|---|
| Combination* | First part | A (1.0) | C (0) | B (2.0) | A (1.0) | C (0) |
| | Second part | B (0) | A (1.0) | B (0) | A (1.0) | B (0) |
| Split hair-generation ratio (%)<br>Prevention of moisture loss | | 30<br>A | 32<br>A | 26<br>A | 22<br>A | 58<br>C |
| *: The numeral in parentheses means the amount of the diamide compound (wt.%). | | | | | | |

It is apparent from the above-described evaluation results that the use of the diamide compound (1) provides good split-hair suppressing effects and moisture-loss preventing effects even when it is incorporated in either the first part or second part of the perming agent; and the effect is proportional to the total amount of the diamide compounds (1) incorporated in the first part and second part.

Example 5: Permanent Waving Composition

| First part composition | (wt.%) |
|---|---|
| Diamide compound (D) | 1.5 |
| Ammonium thioglycolate (50 wt.%) | 12.0 |
| Ammonium bicarbonate | 2.5 |
| Ethanol | 5.0 |
| Propylene glycol | 15.0 |
| Disodium Edetate | 0.5 |
| Polyoxyethylene (20) cetyl ether | 1.0 |
| Ammonia (28 wt.%) | Amount to adjust the pH to 9.0 |
| Perfume | q.s. |
| Purified water | Balance |
| Total | 100.0 |

| Second part composition | (wt.%) |
|---|---|
| Sodium bromate | 7.5 |
| Propylene glycol | 5.0 |
| Polyoxyethylene (20) cetyl ether | 0.75 |
| Polyoxyethylene (2) cetyl ether | 0.25 |
| Amino-modified silicone emulsion ("SM8704C", product of Dow Corning Toray Silicone) | 0.5 |
| Keratin hydrolysate ("Promois WK-H" product of Seiwa Kasei) | 0.5 |
| Purified water | Balance |
| Total | 100.0 |

Example 6: Permanent Waving Composition

| First part composition | (wt.%) |
|---|---|
| Diamide compound (F) | 1.0 |
| Ammonium thioglycolate (50 wt.%) | 1.5 |
| L-cysteine | 4.5 |
| Diammonium dithiodiglycolate | 0.5 |
| Ethanol | 5.0 |

(continued)

| First part composition | (wt.%) |
|---|---|
| Propylene glycol | 10.0 |
| Disodium Edetate | 0.5 |
| Polyoxyethylene (20) cetyl ether | 1.0 |
| Monoethanolamine | Amount to adjust the pH to 9.0 |
| Perfume | q.s. |
| Purified water | Balance |
| Total | 100.0 |

| Second part composition | (wt.%) |
|---|---|
| Diamide compound (F) | 1.0 |
| Sodium bromate | 8.0 |
| Propylene glycol | 12.0 |
| Ethanol | 3.0 |
| Polyoxyethylene (20) cetyl ether | 0.75 |
| Polyoxyethylene (2) cetyl ether | 0.25 |
| Amino-modified silicone emulsion ("SM8704C", product of Dow Corning Toray Silicone) | 0.5 |
| Keratin hydrolysate ("Promois WK-H" product of Seiwa Kasei) | 0.5 |
| Purified water | Balance |
| Total | 100.0 |

Example 7: Curl straightener composition

The first part and second part having the below-described compositions, respectively, were mixed in equal amounts and provided for use.

| First-part composition | (wt.%) |
|---|---|
| Diamide compound (F) | 1.5 |
| Ammonium thioglycolate (50 wt.%) | 13.0 |
| Ammonium bicarbonate | 2.0 |
| β-naphthalensulfonic acid | 5.0 |
| 2-Benzyloxyethanol | 3.5 |
| Ethanol | 10.0 |
| Propylene glycol | 5.0 |
| Disodium Edetate | 0.5 |
| Monoethanolamine | Amount to adjust the pH to 9.0 |
| Perfume | q.s. |
| Purified water | Balance |
| Total | 100.0 |

| Second part composition | (wt.%) |
|---|---|
| Diamide compound (F) | 1.5 |
| Hydrogen peroxide (35 wt.%) | 5.0 |
| Lactic acid | 4.5 |
| β-Naphthalenesulfonic acid | 1.5 |
| 2-Benzyloxyethanol | 3.5 |
| Ethanol | 10.0 |

(continued)

| Second part composition | (wt.%) |
|---|---|
| Polyoxyethylene (20) cetyl ether | 1.0 |
| Sodium hydroxide | Amount to adjust the pH to 3.5 |
| Purified water | Balance |
| Total | 100.0 |

Example 8: Hair straightener composition (aerosol foam type; not within the scope of claim 1)

A stock solution and a propellant each having the below-described composition were filled in a predetermined aerosol container at a stock solution:propellant weight ratio of 85:15.

| Stock solution | (wt.%) |
|---|---|
| Diamide compound (G) | 2.0 |
| Sodium bisulfite | 1.0 |
| 2-Benzyloxyethanol | 3.0 |
| Ethanol | 10.0 |
| 1,3-Butylene glycol | 5.0 |
| Polyether-modified silicone ("KF-6005", product of Shin'etsu Chemical) | 0.5 |
| Lactic acid | Amount to adjust the pH to 3.5 |
| Perfume | q.s. |
| Purified water | Balance |
| Total | 100.0 |

| Propellant | (wt.%) |
|---|---|
| LPG (3.5 kg/cm$^2$) | 85.0 |
| Dimethyl ether | 15.0 |
| Total | 100.0 |

Any of the compositions obtained in Examples 5 to 8 caused markedly little hair damage and did not impair the moistness of the hair.

## Claims

1. A method of permanent waving or permanent straightening hair to a desired shape comprising:

    i) applying to hair a first part composition comprising a reducing agent, and then
    ii) applying to hair a second part composition comprising an oxidizing agent **characterized in that** a diamide compound represented by the following formula (1) is present in the first part composition, the second part composition or in both compositions:

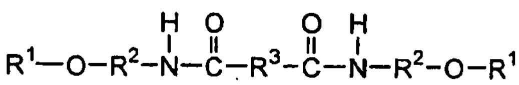

(1)

wherein, $R^1$ represents a linear or branched $C_{1-12}$ hydrocarbon group which may be substituted by a hydroxy and/or alkoxy group, $R^2$ represents a linear or branched divalent $C_{1-5}$ hydrocarbon group, and $R^3$ represents a linear or

branched divalent $C_{1-22}$ hydrocarbon group.

**2.** Method of claim 1, wherein the diamide compound (1) has the following formula (F):

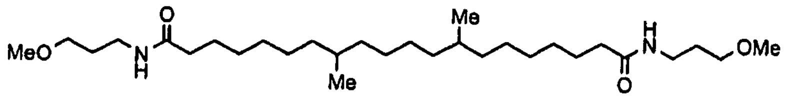

(F)

## Patentansprüche

**1.** Verfahren zum Dauerwellen oder permanenten Begradigen von Haar in eine gewünschte Form, umfassend

(i) Auftragen einer ersten Teilzusammensetzung, umfassend ein Reduktionsmittel, auf das Haar und anschließendes
(ii) Auftragen einer zweiten Teilzusammensetzung, umfassend ein Oxidationsmittel, auf das Haar, **dadurch gekennzeichnet, dass** eine Diamidverbindung mit der folgenden Formel (1) in der ersten Zusammensetzung, der zweiten Zusammensetzung oder in beiden Zusammensetzungen vorhanden ist:

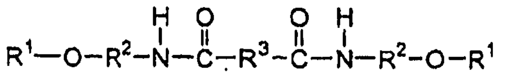

(1)

worin $R^1$ eine lineare oder verzweigte $C_{1-12}$-Kohlenwasserstoffgruppe ist, die durch eine Hydroxy- und/oder Alkoxygruppe substituiert sein kann, $R^2$ eine lineare oder verzweigte bivalente $C_{1-5}$-Kohlenwasserstoffgruppe ist und $R^3$ eine lineare oder verzweigte, bivalente $C_{1-22}$-Kohlenwasserstoffgruppe ist.

**2.** Verfahren nach Anspruch 1, worin die Diamidverbindung (1) die folgende Formel (F) aufweist:

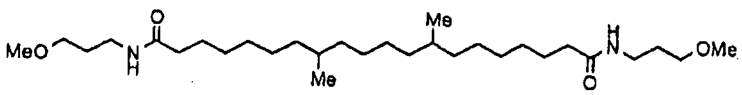

(F)

## Revendications

**1.** Procédé d'ondulation permanente ou de défrisage permanent des cheveux en une forme souhaitée comprenant :

i) l'application aux cheveux d'une composition de première partie comprenant un agent réducteur, et puis
ii) l'application aux cheveux d'une composition de seconde partie comprenant un agent oxydant **caractérisé en ce que** un composé diamide représenté par la formule (1) suivante est présent dans la composition de première partie, la composition de seconde partie ou dans les deux compositions :

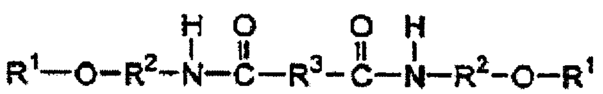

(1)

dans laquelle, $R^1$ représente un groupe hydrocarboné en $C_{1-12}$ linéaire ou ramifié, qui peut être substitué par un groupe hydroxy et/ou alcoxy, $R^2$ représente un groupe hydrocarboné en $C_{1-5}$ divalent linéaire ou ramifié, et $R^3$

représente un groupe hydrocarboné en $C_{1-22}$ divalent linéaire ou ramifié.

**2.** Procédé selon la revendication 1, dans lequel le composé diamide (1) possède la formule (F) suivante:

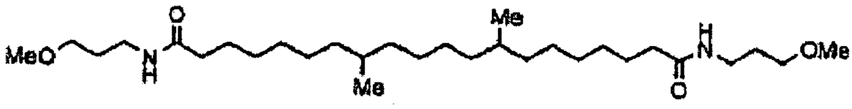

(F)